# EUROPEAN PATENT APPLICATION

(11) **EP 4 527 829 A1**
(43) Date of publication of application: **26.03.2025**
(21) Application number: 23198578.9
(22) Date of filing: 20.09.2023
(51) Int. Cl.: C07C 51/09, C07C 51/41, C07C 51/43, C07C 67/14, C07C 53/21, C07C 69/63, C07C 201/00

(54) **PROCESS FOR THE PURIFICATION OF PERFLUOROALKYL ACIDS**

(71) Applicant: Chiron AS, 7041 Trondheim (NO)
(72) Inventor: Liu, Huiling, 7049 Trondheim (NO)
(74) Representative: Meissner Bolte Düsseldorf Patentanwälte Rechtsanwälte Partnerschaft mbB

(57) **Abstract**

The present invention is directed to poly- and perfluoroalkyl sulfonic acids and poly- and perfluoroalkyl acids and processes for the purification and isolation of isomers from mixtures as well as highly pure poly- and perfluoroalkyl sulfonic acids and poly- and perfluoroalkyl acids and the corresponding uses.

## Description

The present invention is directed to poly- and perfluoroalkyl acids and their derivatives and processes for the purification and isolation of isomers from mixtures as well as highly pure poly- and perfluoroalkyl acids and the corresponding uses.

Any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as set forth herein supersedes any conflicting material incorporated herein by reference. Any material, or portion thereof, that is said to be incorporated by reference herein, but which conflicts with existing definitions, statements, or other disclosure material set forth herein is only incorporated to the extent that no conflict arises between that incorporated material and the existing disclosure material.

### Prior Art:

Poly- and perfluoroalkyl substances (PFAS), a class of artificially manufactured highly fluorinated aliphatic compounds, have been extensively used in various industrial and commercial products. PFAS (CₙF₂ₙ₊₁-R; R: functional group) comprised of a hydrophobic fluorinated alkyl chain at one end and a hydrophilic functional group at the other. Owing to the strong C-F bonds, PFAS have high thermal and chemical stability. This unique combination of physical and chemical properties, along with the hydrophobic and lipophobic moieties, make PFAS are long-lived substances with the ability to repel both water and oil, PFAS are extremely useful in a range of variety of applications and products e.g. textiles, paper, packaging materials, paint and varnish, and fire-extinguishing liquids. The general purpose of treating consumer products with PFAS is to impart an extended service life and durability. This practice, in turn, results in prolonged exposure of humans, wildlife, and the environment to PFAS. PFAS are extremely resistant to chemical hydrolysis, photolysis and biodegradation and therefore highly persistent in the environment.

Today, more than 4000 PFAS are manufactured and used world-wide, including both conventional (e.g., C8-based PFOA and PFOS) and alternative, new emerging PFAS (short-chain PFAS and newly identified fluorinated replacements of conventional PFAS). Anticipating long-term exposure effects due to their persistent and bio-accumulative nature, conventional PFAS were voluntarily phased out due to regulations. Still, high levels of PFAS can be found in the environment.

Many environmental samples contain both branched and linear isomers (n-isomers) of PFAS, and the mixture of linear and branched isomers presents challenges in providing an accurate quantification of many PFAS in environmental matrices. Isomer-specific analysis of PFAS is difficult and methods, analytical reference standards of high quality and fully characterized for isomer-specific identification and purity are still a requirement for the accurate and adequate analysis of PFAS. Another interest in being able to identify and quantity individual isomers present in commercial PFAS arises from the possibility that different isomers may exhibit differences in toxicity. PFAS compounds as fine chemicals in gram quantities are commercially available However, these compounds are as a general rule the products from the high volume production methods industrially. They are generally of variable quantity and impurities such as congeners, homologues, polymers, and ash, are generally never pure n-isomers due to the high-volume production process. Moreover, PFAS compounds are hydroscopic and they may contain +/- 10 % water. Therefore, the commercial PFAS products are not suitable as for making reference materials or even certified reference materials without further purification and relevant analysis. Despite of that they are used by several reference materials producers without further elaboration.

Conventional purification techniques such as flash column chromatography, distillation and recrystallization are normally used for the purification of PFAS compounds in order to remove the impurities such as inorganic salts, ash, polymer impurities and water contaminant, but PFAS isomers and also possible homologues cannot be separated directly by these techniques and the available PFAS therefor only have a limited isomerical purity.

Only few documents, like US 2,920,086 or J. Org. Chem. 1959, 24 (2), 246-247, relate to some kind of purification of PFAS but none of them discloses ways to achieve isomerically pure PFAS.

Accordingly, there still is a need for isomerically pure PFAS of higher purity as well as for methods to obtain them.

### Object of the invention:

It was an object of the present invention to provide a process for the provision of isomerically pure n-isomers of either poly- and perfluoroalkyl sulfonic acids or poly-and perfluoroalkyl carboxylic acids.

It was a further object of the invention to provide isomerically pure perfluoroalkyl sulfonic acids or perfluoroalkyl carboxylic acids.

It was a further object of the invention to provide isomerically pure derivatives of perfluoroalkyl sulfonic acids or perfluoroalkyl carboxylic acids.

Additionally, it was an object of the present invention to provide corresponding uses for these perfluoroalkyl sulfonic acids or perfluoroalkyl carboxylic acids.

Further objects will become apparent to those skilled in the art upon regarding the following disclosure.

### Solution:

These and other objects are solved by the matter outlined in the appended independent claims.

Preferred embodiments are outlined in the dependent claims as well as the following description.

### Detailed description:

It should be understood that this invention is not limited to the embodiments disclosed in the description that follows, but is intended to cover modifications that are within the spirit and scope of the invention, as de-fined by the claims.

In the present application, including the claims, other than in the operating examples or where otherwise indicated, all numbers expressing quantities or characteristics are to be understood as being modified in all instances by the term "about". Accordingly, unless indicated to the contrary, any numerical parameters set forth in the following description may vary depending on the desired properties one seeks to obtain in the compositions and methods according to the present disclosure. At the very least, and not as an attempt to limit the application of the doctrine of equivalents to the scope of the claims, each numerical parameter described in the present description should at least be construed in light of the number of reported significant digits and by applying ordinary rounding techniques.

Also, it should be understood that any numerical range recited herein is intended to include all sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10. The terms "one," "a," or "an" as used herein are intended to include "at least one" or "one or more," unless otherwise indicated.

In the present invention, unless otherwise specified, the reactions are conducted at room temperature, which is understood as being 20°C.

In the present invention, unless otherwise specified, the reactions are conducted at ambient pressure, which is understood as being 101,325 kPa.

In the context of the present invention, "isomerically pure" means that the isomerical purity of the respective compound is at least 97% chromatographic purity, preferably at least 98% more preferably at least 99% and in particular at least 99.5%

The present invention in a first main embodiment is directed to a process for the provision of isomerically pure n-isomers of either
a) poly- and perfluoroalkyl sulfonic acids of the general formula (I)

   CₘF₂ₘ₊₁₋ₚHₚ-SO₂-OH,

   or
b) poly- and perfluoroalkyl carboxylic acids of the general formula (II)

   CₙF₂ₙ₊₁₋ₚHₚ-CO-OH,

   wherein
   m is an integer of at least 4, preferably 4 to 24, more preferably 4 to 18, even more preferably 4 to 12, in particular 8,
   n is an integer of at least 3, preferably 3 to 23, more preferably 3 to 17, even more preferably 3 to 11, in particular 7,
   p is 0 or 1 to ≤m, preferably 0 or 1 to 4,
   comprising the following steps or consisting thereof:
      I) providing a mixture, in particular a mixture of isomers, of
         poly- and/or perfluoroalkyl sulfonic acids, esters thereof or acid fluorides thereof, poly- and/or preferably perfluoroalkyl sulfonic acids or acid halogenides, in particular fluorides, thereof,
            or
         poly- and/or perfluoroalkyl carboxylic acids, esters thereof or acid fluorides thereof, preferably poly- and/or perfluoroalkyl carboxylic acids or acid halogenides, in particular chlorides or fluorides, thereof,
            or
         poly- and/or perfluoroalkyl sulfonic acids, esters thereof or acid fluorides thereof, preferably poly- and/or perfluoroalkyl sulfonic acids or acid halogenides, in particular fluorides, thereof, and poly- and/or perfluoroalkyl carboxylic acids, esters thereof or acid fluorides thereof, preferably poly- and/or perfluoroalkyl carboxylic acids or acid halogenides, in particular chlorides or fluorides, thereof;
      II) converting the provided mixture into (usually a crystalline solid of) either
      IIa) aryl esters, or
      IIb) ammonium salts;
      III) separating the respective products of step II) by successive recrystallization into fractions with desired isomer of >97% chromatographic purity, preferably from a solvent or solvent mixture selected from the group consisting of methanol, ethanol, ethanol/water, ethanol/diethyl ether, diethyl ether, ethyl acetate, hexane, isooctane and further preferably repeating the recrystallization several times;
      IV) selecting at least one product fraction from those obtained in step III) and converting the at least one selected product fraction either by
      IVa) hydrogenation of the aryl esters to form the respective isomer of poly- and/or perfluoroalkyl sulfonic acid, or poly- and/or perfluoroalkyl carboxylic acid,
         or
      IVb) acidification of the ammonium salts to form the respective isomer of poly-and/or perfluoroalkyl sulfonic acid or poly- and/or perfluoroalkyl carboxylic acid.

A preferred one of the above alternatives is directed to a process for the provision of isomerically pure n-isomers of either
a) perfluoroalkyl sulfonic acids (PFSA) of the general formula (Ia)

   CₘF₂ₘ₊₁-SO₂-OH,

   i.e. an n-isomer, or
b) perfluoroalkyl carboxylic acids (PFCA) of the general formula (IIa)

   CₙF₂ₙ₊₁-CO-OH,

   i.e. an n-isomer, with n and m being as defined above.

As this alternative is directed to the perfluorinated compounds, only, the formulae (I) and (II) were simplified to (Ia) and (IIa) by omitting p, which is zero for perfluorinated compounds.

In a further optional embodiment, the present invention also encompasses an additional step V) in which the product obtained in IVa) or IVb) is further purified according to techniques known in the art, however, in the context of the present invention this i usually not necessary and hence optional.

In a preferred embodiment of the present invention the reaction of step IIa) proceeds according to the
general scheme (1)

   CₘF₂ₘ₊₁₋ₚHₚ-SO₂-X + Ar-OH → CₘF_{zm+1-p}Hₚ-SO₂-OAr,

   or simplified, for the perfluorinated compounds only, general scheme (1a)

   CₘF₂ₘ₊₁-SO₂-X + Ar-OH → CₘF₂ₘ₊₁-SO₂-OAr,

   or
general scheme (2)

   CₙF₂ₙ₊₁₋ₚHₚ-CO-Y + Ar-OH → CₙF₂ₙ₊₁₋ₚHₚ-CO-OAr,

   or simplified, for the perfluorinated compounds only, general scheme (2a)

   CₙF₂ₙ₊₁-CO-Y + Ar-OH → CₙF₂ₙ₊₁-CO-OAr,
wherein
m is an integer of at least 4, preferably 4 to 24, more preferably 4 to 18, even more preferably 4 to 12, in particular 8,
n is an integer of at least 3, preferably 3 to 23, more preferably 3 to 17, even more preferably 3 to 11, in particular 7,
p is 0 or 1 to ≤m, preferably 0 or 1 to 4,
X is hydroxyl, fluorine or chlorine,
Y is hydroxyl, fluorine or chlorine, and
Ar is a substituted or non-substituted aryl residue, preferably selected from the group consisting of substituted or non-substituted 1-naphthyl, substituted or non-substituted 2-naphthyl, heterocyclic naphthyls, in particular substituted or non-substituted 2-quinoliny or 8-quinolinyl, poly phenyls such as 4-phenylphenyl. With this the aryl esters are formed as crystalline solids. The substituents are preferably selected from the group consisting of alkyl, especially C₁-C₆-akyl, alkyloxy, especially C₁-C₆-akyloxy, halogen, -CN, -NH₂, -NOz and mixtures thereof. In some embodiments the substituents with higher polarity are preferred. The compounds, if substituted may be fully substituted or mono- or di-substituted, preferably mono-substituted.

It should be noted that in principle Ar may also be phenyl, in some embodiments fully substituted or mono- or di-substituted, preferably mono-substituted, with substituents selected from the group consisting of -CN, -OCH₃, -NOz and mixtures thereof. However, as phenyl esters are oily substances, their handling is more cumbersome and therefore not preferred (but still possible).

In some embodiments of the present invention, in the reactions of schemes (1), (1a), (2) and/or (2a) the acids themselves are used as starting point and in some embodiments the sulfonyl fluorides/chlorides or carboxyl fluorides, carboxyl chlorides are used.

As it can be difficult to make the esters from sulfonic acids directly, it is sometimes preferred to use poly- and/or perfluoroalkyl sulfonyl fluoride for the above reaction. Similarly, also PFCA esters can be synthesized either from carboxyl fluoride or chloride,

In some embodiments it is preferred, if the PFCA esters are bulky on the aryl group, in order to prevent them from being present as oily compounds instead of solid/crystalline. In this context, the term "bulky" is preferable understood to mean that the aryl group is bigger than a phenyl group, preferably fused two or three rings either fused benzene rings like naphthalene or phenanthrene, or biphenyl or terphenyl.

In a further preferred embodiment of the present invention in this context, these reactions (i.e. according to scheme (1)/(1a) or (2)/(2a)) are conducted in an organic solvent or solvent mixture, preferably organic ethers and/or organochlorine compounds, more preferably selected from the group consisting of dimethyl ether, diethyl ether, tetrahydrofuran (THF), dioxane, dimethoxyethane, dichloromethane, chloroform and mixtures thereof, and in the presence of a base, preferably selected from the group consisting of triethyl amine, sodium hydride and pyridine.

In this context, the organochlorine compounds are preferably dichloromethane and/or chloroform.

In this context, the poly- and/or perfluoroalkylsulfonic acid esters from substituted or non-substituted naphthols can for example (in a specific embodiment, but not exclusively, e.g. example 1, below) be obtained by reacting the phenols with perfluoroalkyl sulfonyl fluoride in the presence of triethylamine in ether. Similarly, the poly- and/or perfluoroalkylcarboxylic acid esters from substituted or non-substituted biphenylphenol can for example (in a specific embodiment, but not exclusively, e.g. example 2, below) be obtained by reacting the phenols with perfluoroalkyl carbonyl chloride in the presence of triethylamine or pyridine in dichloromethane or THF. Also in this context, the esters of the heterocyclic phenols can for example (in a specific embodiment, but not exclusively) be prepared by converting the phenols to their sodium salts in the presence of sodium hydride and then reacting the phenol sodium salt with poly- and/or perfluoroalkylsulfonyl fluoride (or chloride) in dimethoxyethane. This method is especially recommended when the substrate is poorly soluble in ether.

It should be noted that there are also other ways to obtain the compounds, and not only the just mentioned examples, which would be readily known to the person skilled in the art.

The poly- and/or perfluoroalkylsulfonic acid esters are then purified by, preferably repeated, recrystallization to remove, inter alia, (unwanted) isomers and homologues. This can in some particularly preferred examples be done with a solvent selected from the group consisting of methanol, ethanol, diethyl ether, hexane, isooctane and mixtures thereof.

In another preferred embodiment of the present invention the reaction of step IIb) proceeds according to the
general scheme (3)

   CₘF₂ₘ₊₁₋ₚHₚ-SO₃H + NR¹R²R³ → CₘF₂ₘ₊₁₋ₚHₚ-SO₃⁻ N⁺HR¹R²R³,

   or simplified, for the perfluorinated compounds only, general scheme (3a)

   CₘF₂ₘ₊₁-SO₃H + NR¹R²R³ → CₘF₂ₘ₊₁-SO₃⁻ N⁺HR¹R²R³,

   or
general scheme (4)

   CₙF₂ₙ₊₁₋ₚHₚ-CO₂H + NR¹R²R³ → CₙF₂ₙ₊₁₋ₚHₚ-CO₂⁻ N⁺HR¹R²R³,

   or simplified, for the perfluorinated compounds only, general scheme (4a)

   CₙF₂ₙ₊₁-CO₂H + NR¹R²R³ → CₙF₂ₙ₊₁-CO₂⁻ N⁺HR¹R²R³,
wherein R¹, R² and R³ are, each independently of each other, hydrogen or an alkyl group with at least five carbon atoms, or R¹, R² and R³ together with the nitrogen can form a cycloalkyl amine or heterocyclic aromatic amine, and
m is an integer of at least 4, preferably 4 to 24, more preferably 4 to 18, even more preferably 4 to 12, in particular 8,
n is an integer of at least 3, preferably 3 to 23, more preferably 3 to 17, even more preferably 3 to 11, in particular 7,
p is 0 or 1 to ≤m, preferably 0 or 1 to 4.

The alkyl groups in preferred embodiments have at least five carbon atoms and at most 24 carbon atoms.

It should be noted, that while it is preferred to employ PFSA and PFCA themselves in the context of schemes (3) and (4), it is also possible to employ the sulfonyl chlorides/fluorides or carboxyl chlorides/fluorides as well. However, the use of the acids themselves is usually preferred because of ease of procedure.

In preferred embodiments, the amines employed here should be bulky, should contain a ring or in a ring or contain one or two long alkyl chains. In this context, the term "bulky" is preferable understood to mean that at least one of R¹, R² and R³ is an alkyl which has at least four carbon atoms; preferably two of them or in particular all three of them are alkyl residues with at least four carbon atoms each, or in other preferred embodiments at least two of those form a ring comprising at least four carbon atoms and optionally incorporate the nitrogen, wherein the ring can be aromatic, aliphatic or anti-aromatic, preferably hetero-aliphatic or hetero-aromatic.

In a further preferred embodiment of the present invention in this context (i.e. reactions according to scheme (3)/(3a) or (4)//4a)) the amine NR¹R²R³ is selected from the group consisting of primary amines, secondary amines, tertiary amines, and mixtures thereof, the amine being particularly preferably selected from the group consisting of pyrrolidine, piperidine, piperazine, N-phenyl-piperazine, pyridine and mixtures thereof and the reactions are conducted in an organic solvent or solvent mixture, which is preferably selected from the group consisting of acetone, isopropanol and mixtures thereof.

In this context, the poly- and/or perfluoroalkylcarboxylic acid salts can for example (in a specific embodiment, but not exclusively, e.g. example 3, below) be obtained by reacting the amines with poly- and/or perfluoroalkanecarboxylic acids at 0-5°C in 2-propanol. Similarly, the poly- and/or perfluoroalkylsulfonic acid salts can for example (a specific one in a specific embodiment, but not exclusively, e.g. example 4, below) be obtained by reacting the amines with poly- and/or perfluoroalkanesulfonic acids in 1:1 equivalent at room temperature in acetone. The salts produced in these examples can, if desired, then be filtered at that temperature.

Additionally, in some other preferred embodiments of the present invention, step IVa) is a hydrogenation with hydrogen gas in the presence of a hydrogenation catalyst, in particular palladium supported on charcoal or activated carbon, and the solvent for the reaction is an organic solvent, preferably selected from the group consisting of methanol, ethanol, n-propanol, iso-propanol, n-butanol, iso-butanol and mixtures thereof.

In some specific embodiments of the present invention, the n-isomers of the poly-and/or perfluoroalkylsulfonic acid esters or poly- and/or perfluoroalkylcarboxylic acid esters obtained after purification are hydrogenated at room temperature in methanol as solvent over palladium/charcoal as catalyst to give the n-isomers of poly- and/or perfluoroalkylsulfonic acid or poly- and/or perfluoroalkylcarboxylic acid.

In some alternative preferred embodiments of the present invention, step IVb) is an acidification with an organic or inorganic acid or a mixture of organic acids or a mixture of inorganic acids or a mixture of organic and inorganic acids, in particular hydrochloric acid and/or sulphonic acid as inorganic acids or p-toluenesulfonic acid as organic acid. Preferred are inorganic acids, in particular hydrochloric acid and/or sulphonic acid. The acids can usually be employed in concentrations of 0.5 mol/l to 5 mol/l, preferably 1 mol/l to 3 mol/l, in particular 1 mol/l.

In some specific embodiments of the present invention, the n-isomers of the poly-and/or perfluoroalkylsulfonic acid salts or poly- and/or perfluoroalkycarboxylic acid salts obtained after purification are treated with hydrochloride (1 mol/l solution in water) at room temperature in water to give the n-isomers of poly- and/or perfluoroalkylsulfonic acid or poly- and/or perfluoroalkylcarboxylic acid.

Also some preferred embodiments of the present invention are characterized in that the product fraction selected in step IV) is a fraction containing or consisting mainly of a specific n-isomer. In this context it is to be understood, that "containing or consisting mainly of" means that the amount of the n-isomer in the selected product fraction is at least 90% of chromatographic purity, preferably at least 95%, particularly preferably at least 97%.

It is especially preferred in the context of the present invention, if the process according to the invention is directed to obtaining either poly- and/or perfluoro-n-octyl sulfonic acid, or poly- and/or perfluoro-n-octyl carboxylic acid, in particular perfluoro-n-octyl sulfonic acid (PFOS), or perfluoro-n-octyl carboxylic acid (PFOA), in step IV).

A further main embodiment of present invention directed to
perfluoroalkyl sulfonic acids of the general formula (Ia)

   CₘF₂ₘ₊₁SO₂-OH;
perfluoroalkyl carboxylic acids of the general formula (IIa)

   CₙF₂ₙ₊₁-CO-OH;
aryl esters of perfluoroalkyl sulfonic acids of the general formula (IIIa)

   CₘF₂ₘ₊₁- SO₂-O-Ar;
aryl esters of perfluoroalkyl carboxylic acids of the general formula (IVa)

   CₙF₂ₙ₊₁-CO-O-Ar;
ammonium salts of perfluoroalkyl sulfonic acids of the general formula (Va)

   CₘF₂ₘ₊₁- SO₂O⁻N⁺HR¹R²R³;
ammonium salts of perfluoroalkyl carboxylic acids of the general formula (VIa)

   CₙF₂ₙ₊₁-COO⁻N⁺HR¹R²R³;
in particular,
   - perfluoroalkyl sulfonic acids of the general formula (I) CₘF₂ₘ₊₁- SO₂-OH,
      and
   - perfluoroalkyl carboxylic acids of the general formula (II) CₙF₂ₙ₊₁-CO-OH, wherein m is an integer of at least 4, preferably 4 to 24, more preferably 4 to 18, even more preferably 4 to 12, in particular 8; n is an integer of at least 3, preferably 3 to 23, more preferably 3 to 17, even more preferably 3 to 11, in particular 7.

This main embodiment is especially characterized in that the isomerical purity of these compounds is at least 97% of chromatographic purity, preferably at least 98% more preferably at least 99% and in particular at least 99.5%.

Among these compounds of high isomerical purity according to general formulae (I) and (II), the following are specifically preferred:
- perfluoro-n-butyl sulfonic acid,
- perfluoro-n-pentyl sulfonic acid,
- perfluoro-n-hexyl sulfonic acid,
- perfluoro-n-heptyl sulfonic acid,
- perfluoro-n-octyl sulfonic acid,
- perfluoro-n-nonyl sulfonic acid,
- perfluoro-n-decyl sulfonic acid,
- perfluoro-n-undecyl sulfonic acid,
- perfluoro-n-dodecyl sulfonic acid,
- perfluoro-n-tridecyl sulfonic acid,
- perfluoro-n-tetradecyl sulfonic acid,
- perfluoro-n-pentadecyl sulfonic acid,
- perfluoro-n-hexadecyl sulfonic acid,
- perfluoro-n-heptadecyl sulfonic acid,
- perfluoro-n-octadecyl sulfonic acid,
- perfluoro-n-butyl carboxylic acid,
- perfluoro-n-pentyl carboxylic acid,
- perfluoro-n-hexyl carboxylic acid,
- perfluoro-n-heptyl carboxylic acid,
- perfluoro-n-octyl carboxylic acid,
- perfluoro-n-nonyl carboxylic acid,
- perfluoro-n-decyl carboxylic acid,
- perfluoro-n-undecyl carboxylic acid,
- perfluoro-n-dodecyl carboxylic acid,
- perfluoro-n-tridecyl carboxylic acid,
- perfluoro-n-tetradecyl carboxylic acid,
- perfluoro-n-pentadecyl carboxylic acid,
- perfluoro-n-hexadecyl carboxylic acid,
- perfluoro-n-heptadecyl carboxylic acid,
- perfluoro-n-octadecyl carboxylic acid.

More preferred are:
- perfluoro-n-hexyl sulfonic acid,
- perfluoro-n-hexyl carboxylic acid,
- perfluoro-n-decyl sulfonic acid,
- perfluoro-n-octyl sulfonic acid,
- perfluoro-n-octyl carboxylic acid.
- perfluoro-n-decyl carboxylic acid,
- perfluoro-n-dodecyl sulfonic acid,
- perfluoro-n-dodecyl carboxylic acid.

Particularly preferred are:
- perfluoro-n-octyl sulfonic acid, and
- perfluoro-n-octyl carboxylic acid.

An even further main embodiment of present invention directed to the uses of the isomerically pure poly- and perfluoroalkyl sulfonic acids or isomerically pure poly- and perfluoroalkyl carboxylic acids obtained in a process according to the present invention as reference materials or internal standards, preferably for analytical processes, in particular in the analysis and/or examination of poly- or perfluoroalkyl substances or products containing them.

The present invention in this context is also directed to the use of the isomerically pure poly- and/or perfluoroalkyl sulfonic acids of the general formula (I) CₘF₂ₘ₊₁₋ₚHₚ-SO₂-OH, and poly- and/or perfluoroalkyl carboxylic acids of the general formula (II) CₙF₂ₙ₊₁₋ₚHₚ-CO-OH, in particular perfluoroalkyl sulfonic acids of the general formula (Ia) CₘF₂ₘ₊₁- SO₂-OH, and poly- and/or perfluoroalkyl carboxylic acids of the general formula (IIa) CₙF₂ₙ₊₁-CO-OH, wherein the variables are as defined above, as reference materials or internal standards, preferably for analytical processes, in particular in the analysis and/or examination of poly- or perfluoroalkyl substances or products containing them.

It is preferred in the context of these embodiments, if the analytical processes are conducted with a method selected from the group consisting of HPLC, LC, GC, HRMS, ¹⁹F-NMR, ¹H-NMR, ¹³C-NMR and combinations thereof.

One preferred specific embodiment according to the present invention is directed to a process for the provision of isomerically pure perfluoroalkyl sulfonic acids (PFAS) of the general formula (Ia)

CₘF₂ₘ₊₁-SO₂-OH,

wherein m is as defined above,
comprising the following steps or consisting thereof:
   I) providing a mixture of isomers of perfluoroalkyl sulfonyl fluoride, perfluoroalkyl sulfonyl chloride, perfluoroalkyl sulfonic acids or esters thereof, in particular perfluoroalkyl sulfonic acids or esters thereof;
   II) converting the provided mixture into aryl esters as defined above, in particular 2-naphthyl esters;
   III) separating the respective products of step II) by recrystallization into at least two product fractions;
   IV) selecting at least one product fraction from those obtained in step III) and converting the at least one selected product fraction by hydrogenation to form the respective perfluoroalkyl sulfonic acid,
One other preferred specific embodiment according to the present invention is directed to a process for the provision of isomerically pure perfluoroalkyl carboxylic acids (PFCA) of the general formula (IIa)

   CₙF₂ₙ₊₁-CO-OH (II),
wherein n is as defined above,
comprising the following steps or consisting thereof:
   I) providing a mixture of isomer, of perfluoroalkanoyl fluorides, perfluoroalkanoyl chlorides, perfluoroalkyl carboxylic acids or esters thereof, in particular perfluoroalkyl carboxylic acids or esters thereof;
   II) converting the provided mixture into ammonium salts, in particular pyrrolidinium salts, piperidinium salts, piperazinium salts, N-phenyl-piperazinium salts, pyridinium salts and mixtures thereof;
   III) separating the respective products of step II) by recrystallization into at least two product fractions;
   IV) selecting at least one product fraction from those obtained in step III) and converting the at least one selected product fraction by acidification to form the respective perfluoroalkyl carboxylic acid;

One particular embodiment of the present invention is accordingly the purification of poly- and perfluoroalkyl sulphonic acids (PFSAs) by recrystallization of aryl esters of the acids according to the above described process.

One other particular embodiment of the present invention is the purification of poly-and perfluoroalkyl carboxylic acids (PFCAs) by recrystallization of nitrogen-containing piperazine and/or pyridine salts of the acids according to the above described process.

Other aspects of the present invention relate to the following isomerically pure polyfluoroalkane sulfonic acids or carboxylic acids
- Poly-fluorinated sulfonic acids and carboxylic acids preferably the following:
   2H,2H-perfluorooctanoic acid; 2H,2H-perfluorodecanoic acid; 2H,2H,3H,3H-perfluoroundecanoic acid; 2H,2H,3H,3H-perfluorododecanoic acid; 2H,2H,3H,3H-perfluorohexanoic acid; 2H,2H,3H,3H-perfluorooctanoic acid; 2H,2H,3H,3H-perfluorononanoic acid; 2H,2H,3H,3H-perfluorodecanoic acid; 2H-perfluoroethanoic acid; 2H- and 3H-perfluoropropanoic acid; 2H- and 4H-perfluorobutanoic acid; 2H- and 5H-perfluoropentanoic acid; 2H and 6H-perfluorohexanoic acid; 2H- and 7H-perfluoroheptanoic acid; 2H- and 8H-perfluorooctanoic acid; 2H- and 9H-perfluorononanoic acid; 2H- and 10H-perfluorodecanoic acid; 2H- and 11H-perfluoroundecanoic acid; 1H,1H,2H,2H-perfluorobutanesulfonic acid; 1H,1H,2H,2H-perfluorohexanesulfonic acid; 1H,1H,2H,2H-perfluorooctanesulfonic acid; 1H,1H,2H,2H-perfluorodecanesulfonic acid; 1H,1H,2H,2H-perfluorododecanesulfonic acid;

Other aspects of the present invention relate to the following derivatives of isomerically pure poly- and perfluoroalkane sulfonic acids or carboxylic acids
a) PFAS esters of telomeric alcohols;
b) PFAS allylic alcohol telomers;
c) PFAS telomeric olefins;
d) poly- and perfluoroalkyl N-alkylsulfonamides such as those of the following general formula:

   CₘF₂ₘ₊₁₋ₚH-SO₂-NHR,

   in which R=H, methyl, ethyl, propyl or even higher alkyls;
e) poly- and perfluoroalkyl N-alkyl, N-hydroxyethylsulfonamides such as those of the following general formula:

   CₘF₂ₘ₊₁₋ₚHₚ-SO₂-NR(CH₂CH₂OH),

   in which R=H, methyl, ethyl, propyl or even higher alkyls;
f) poly- and perfluoroalkyl N-alkylsulfonylglycines;
g) poly- and perfluoroalkyl N-alkylsulfonylacrylates;
h) perfluoroalkane N-alkyl, N-hydroxyethylsulfonyl acrylates and methacrylates such as those of the following general formula: 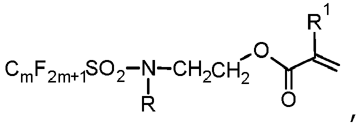 in which R=H, methyl, ethyl, propyl or even higher alkyls and R¹ = H, methyl;
i) PFAS allylic alcohols and telomeric olefins, preferably the following:
   1H,1H,2H,3H-Perfluorohex-2-en-1-ol ; 1H,1H,2H,3H-Perfluoronon-2-en-1-ol ; 1H,1H,2H-Perfluoro-1-octene; 1H,1H,2H-Perfluoro-1-decene,

and mixtures thereof,
wherein
m is an integer of at least 4, preferably 4 to 24, more preferably 4 to 18, even more preferably 4 to 12, in particular 8;
p is 0 or 1 to ≤m, preferably 0 or 1 to 4.

Preferably, a) to i) above are characterized in that their isomerical purity is at least 97 % chromatographic purity, preferably at least 98 % more preferably at least 99 % and in particular at least 99.5 %.

Some of the more important aspects of the present invention can be summarized by way of schemes A and B as follows:
- Pure isomers of poly- and/or perfluoro-n-alkyl sulfonic acids or carboxylic acids via an aryl ester process according to the following scheme A: 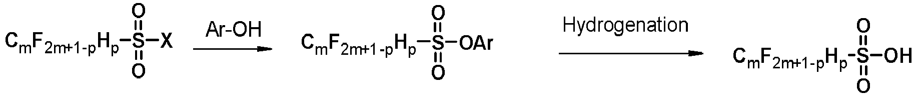 Or 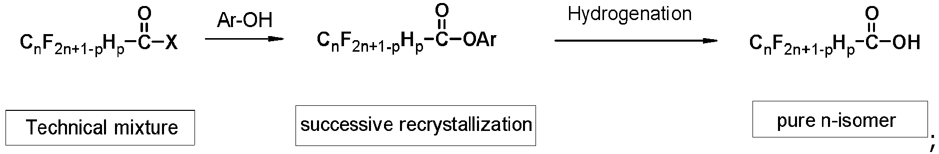
- pure isomers of poly- and perfluoro-n-alkyl sulfonic acids or carboxylic acids via an ammonium salt process according to the following scheme B: 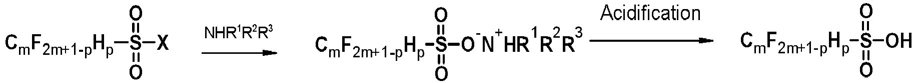 Or 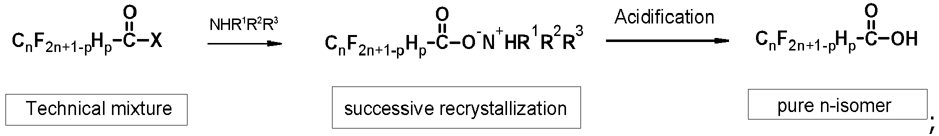 wherein m, n and p are as defined above, in particular m = 4 - 24; n = 3 - 23, p = 0 or p = 1 - 4.

The present invention offers a number of advantages over the prior art, a few of which (but not all) are:
- easily adaptable to the raw compound or compound mixture at hand,
- the process can be performed with operatively relatively easy steps,
- products with exceptional high purity can be obtained.

The invention is now described in more detail with reference to the following non-limiting examples. The following exemplary, non-limiting examples are provided to further describe the embodiments presented herein. Those having ordinary skill in the art will appreciate that variation of these examples are possible within the scope of the invention.

### Examples:

### Example 1 - provision of pure perfluoro-n-octyl sulfonic acid via 2-naphthyl ester

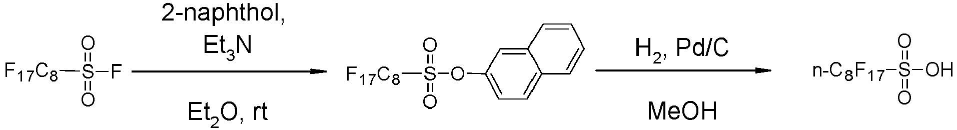

### Step 1 - preparation of 2-naphthyl perfluorooctyl sulfonate ester

Triethylamine (6.89 ml, 49.8 mmol, 1.5 eq) and 2-naphthol (4.78 g, 33.2 mmol, 1 eq) were dissolved in diethyl ether (30 ml) under argon atmosphere. The mixture was cooled to 0°C with an ice-water bath. Commercially available perfluorooctane sulfonyl fluoride with a purity of 82.7% as sum of isomers, n-isomer enriched (25 g, 49.8 mmol, 1.5 eq) was added dropwise and the reaction mixture was warmed to room temperature and then stirred for 18 h. More diethyl ether was added to the reaction, followed by 5% sodium hydroxide solution. The two liquid phases were separated, and the organic phase was washed with water. After drying the organic layer with MgSO₄ the solvent ether was evaporated, the residue is 2-naphthyl perfluorooctanesulfonate as a brown semi-solid mixture, purity 80% by GC-MS analysis.

### Step 2 - purification and isolation of 2-naphthyl perfluoro-n-octanesulfonate

The crude ester product from step 1 was first passed through a short flash chromatography column (Silica, Et₂O/heptane = 1/20) to remove trace of 2-naphthol and polar organic residue. The solvent was then removed and the residue ester crystallized at cooled temperature.

Recrystallization of the solid ester was done with Hexane and diethyl ether to remove branched isomers and homologues, the isolated/enriched n-isomer fraction was recrystallized again with the same solvents, the process was repeated 3-4 times resulting in 2-naphthyl perfluoro-n-octanesulfonate as white solid, 2.6 g, 98.0% (GC-MS) as for the n-isomer.

### Step 3 - preparation of perfluoro-n-octyl sulfonic acid

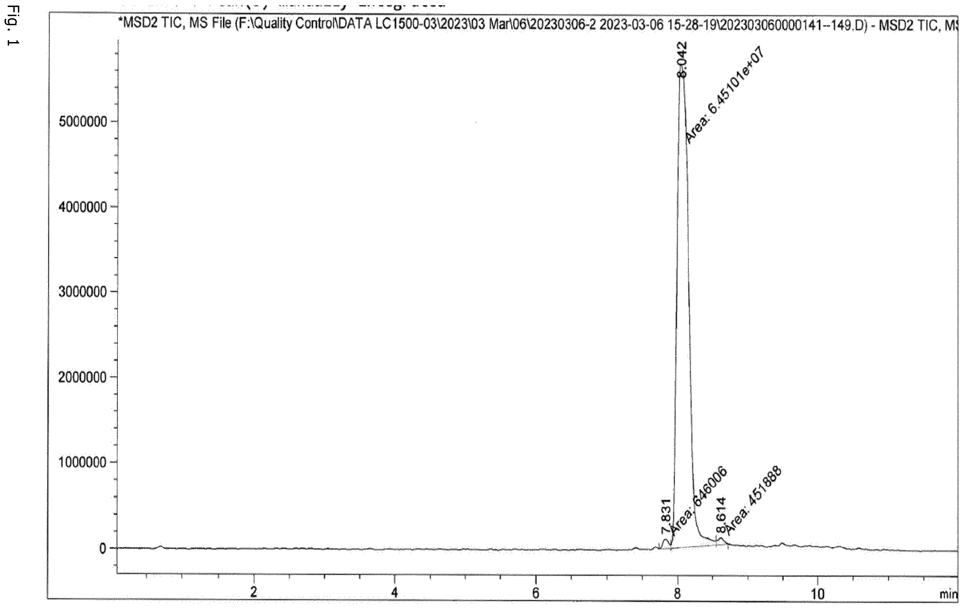

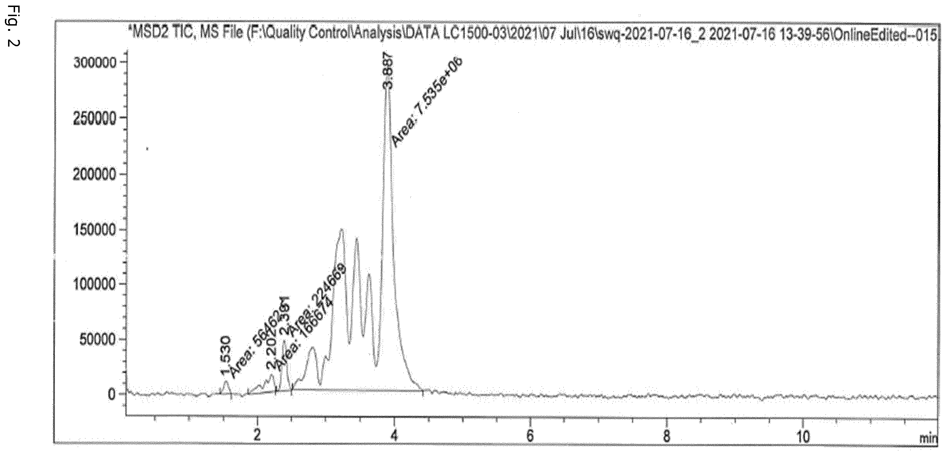

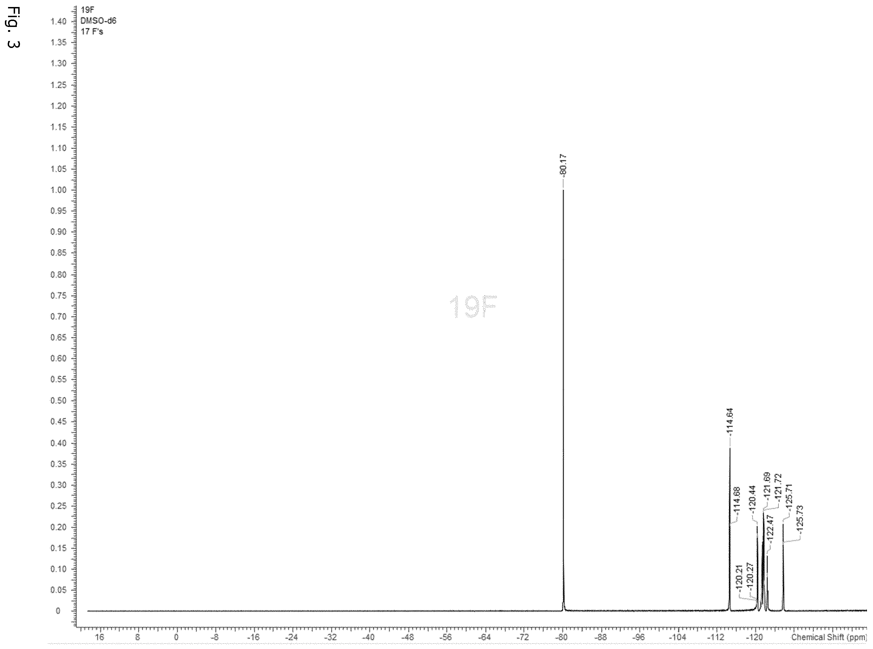

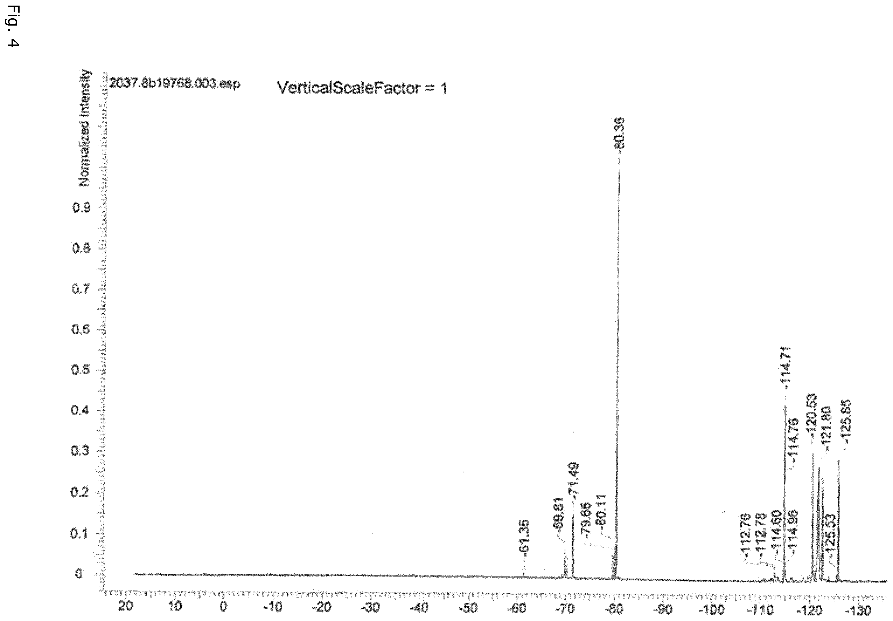

2-naphthyl perfluoro-n-octanesulfonate isolated from the recrystallization step (1.5 g, 2.4 mmol) was dissolved in 350 ml MeOH, 10% Pd/C (0.350 g) was added. The flask was flushed with argon, then hydrogenated at 1 atm (1013 mbar) of hydrogen (balloon) for 2 days at room temperature. The palladium catalyst was filtered off through celite and was washed with MeOH. After the solvent was evaporated, perfluoro-n-octyl sulfonic acid was obtained, which was further purified by flash column chromatography (Silica gel, first 0% to 50% EtOAc in hexane, then 0% to 100% MeOH in dichloromethane (DCM)) to remove impurities from this hydrogenation step. A beige powder product was obtained, weighing 142 mg, purity of 98.5% (HPLC) as for the n-isomer, ¹⁹F-NMR confirmed the structure.
Results - comparison with a commercially available PFOS, technical grade isomer mixture
An LC chromatogram of the prepared n-isomer is reproduced as figure 1.
An LC chromatogram of a commercially available PFOS, technical grade isomer mixture is reproduced as figure 2.
An ¹⁹F-NMR of the prepared n-isomer is reproduced as figure 3.
An ¹⁹F-NMR of a commercially available PFOS, technical grade isomer mixture is reproduced as figure 2.

From comparing the figures it is readily apparent that the present invention leads to much enhanced products having exceptionally high (isomeric) purity.

### Example 2 - provision of pure perfluoro-n-octyl carboxylic acid via p-phenylphenol ester

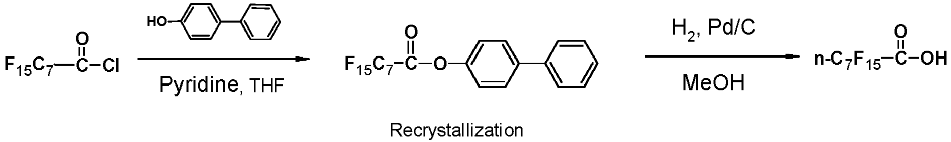

### Step 1 - synthesis of perfluorooctyrate of 4-phenylphenols

To a mixture of p-phenylphenol 1.23 g (7.2 mmol) and pyridine 0,70 g (7,2 mmol) in THF (20 ml) was added perfluorooctyric chloride 3,46 g (8 mmol) in small portions. After the addition was complete, the resulting mixture was allowed to react by heating to reflux with stirring for 3 hours. The pyridine hydrochloride was separated by suction filtration, the filtrate was evaporated to remove THF. The residue was dissolved in chloroform, washed with water and dried over MgSO₄. The solvent was removed to give a solid product weighing 3,51 g.

### Step 2 - purification and isolation of 4-phenylphenol perfluoro-n-octyrate

The crude ester product from step 1 was first passed through a short column (Silica, petroleum ether) to remove traces of the phenol and organic residues. Further purification was done by recrystallization with isooctane to remove branched isomers and homologues. The isolated/enriched n-isomer fraction was recrystallized repeatedly with the same solvents, resulting in 4-phenylphenol perfluoro-n-octyrate as white solid, 1,4 g, 99.5% (GC-MS) as for the n-isomer.

### Step 3 - preparation of perfluoro-n-octanoic acid

4-phenylphenol perfluoro-n-octyrate was hydrogenated in the same way as described in example 1n step3, resulting in perfluoro-n-octanoc acid, 1,1 g, purity of 99.9% (HPLC). The structure is confirmed by ¹⁹F-NMR.

### Example 3 - purification of perfluoro-n-octyl sulfonic acid via pyrrolidinium salt

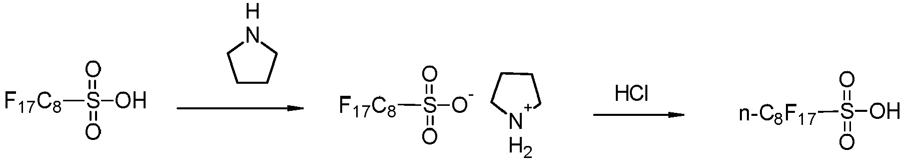

### Step 1 - preparation of pyrrolidium perfluorooctyl sulfonate salt

To a stirred solution of pyrrolidine (4.26 g, 60 mmol) in water (10 ml), perfluorooctanesulfonic acid (25 g, 50 mmol) was added slowly at room temperature. The mixture was warmed to 60°C and stirred overnight. The reaction mixture was washed with diethyl ether (50 ml x 3). Removing the solvent at reduced pressure gave a white solid as the crude perfluorooctylsulfonic acid pyrrolidium salt.

### Step 2 - purification and isolation of pyrrolidium perfluoro-n-octanesulfonate

Recrystallization of the solid salt was done with ethanol/diethyl ether to remove branched isomers and homologues, the isolated/enriched n-isomer fraction was recrystallized repeatedly (3-4 times) with the same solvents, resulting in 2-pyrrolidium perfluoro-n-octanesulfonate as white solid, 26.3 g (46 mmol), 98.8% (HPLC) as for the n-isomer.

### Step 3 - conversion of the salt to perfluoro-n-octyl sulfonic acid

To a solution of pyrrolidium perfluoro-n-octylsulfonate (25 g, 44.7 mmol) in acetone (350 ml) was added HCI (37%, 3.35 ml, 49.2 mmol) at room temperature. The reaction mixture was stirred at room temperature for 1 hour, the mixture was filtered and concentrated to dryness. The residue was diluted with ethyl acetate and washed with water. The organic phase was separated, dried over MgSO₄, and concentrated to afford perfluoro-n-octylsulfonic acid, 21.8 g, 98.0% (HPLC). ¹⁹F-NMR confirmed the structure of n-isomer.

### Example 4 - purification of perfluoro-n-octanoic acid via pyridinium salt

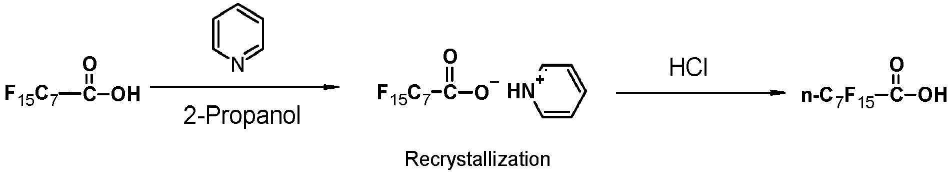

### Step 1 - preparation of pyridinium perfluorooctyrate salt

Perflurooctanoic acid (2.5 g, 5.0 mmol) was mixed in 2-propanol (10 ml), cooled with ice-water bath, pyridine (474.6 mg, 6.0 mmol) was added slowly at the cooled temperature 0-5°C. After the addition was complete, the mixture was heated until the solid dissolved and stirred at 70-80°C for 30 min. The crystals which formed on cooling were suction filtrated and dried to give a white solid as the crude perfluorooctanoic acid pyridinium salt.

### Step 2 - purification and isolation of pyridinium perfluoro-n-octyrate salt

Recrystallization of the solid salt was done with ethanol/diethyl ether to remove branched isomers and homologues, the isolated/enriched n-isomer fraction was recrystallized repeatedly (3-4 times) with the same solvents, resulting in pyridinium perfluoro-n-octyrate as white solid, 2,26 g (4.6 mmol), 98.8% (HPLC) as for the n-isomer.

### Step 3 - conversion of the salt to perfluoro-n-octanoic acid

To a solution of pyridinium perfluoro-n-octyrate (2,0 g, 4.0 mmol) in acetone (50 ml) was added HCI (37%, 0,5 ml) at room temperature. The reaction mixture was stirred at room temperature for 1 hour, the mixture was filtered and concentrated to dryness. The residue was diluted with ethyl acetate and washed with water. The organic phase was separated, dried over MgSO₄, and concentrated to give perfluoro-n-octylsulfonic acid, 1,61 g, 98.7% (HPLC). ¹⁹F-NMR confirmed the structure.

## Claims

1. Process for the provision of isomerically pure n-isomers of either
a) poly- and perfluoroalkyl sulfonic acids of the general formula (I)
CₘF₂ₘ₊₁₋ₚHₚ-SO₂-OH,
or
b) poly- and perfluoroalkyl carboxylic acids of the general formula (II)
CₙF₂ₙ₊₁₋ₚHₚ-CO-OH,
wherein
- m is an integer of at least 4, preferably 4 to 24, more preferably 4 to 18, even more preferably 4 to 12, in particular 8,
- n is an integer of at least 3, preferably 3 to 23, more preferably 3 to 17, even more preferably 3 to 11, in particular 7,
- p is 0 or 1 to ≤m, preferably 0 or 1 to 4,
the process comprising the following steps or consisting thereof:
I) providing a mixture, in particular a mixture of isomers, of poly- or perfluoroalkyl sulfonic acids, esters thereof or acid fluorides thereof,
or
poly- or perfluoroalkyl carboxylic acids, esters thereof or acid fluorides thereof,
or
poly- or perfluoroalkyl sulfonic acids, esters thereof or acid fluorides thereof, and poly- or perfluoroalkyl carboxylic acids, esters thereof or acid fluorides thereof;
II) converting the provided mixture into either
IIa) aryl esters, or
IIb) ammonium salts;
III) separating the respective products of step II) by recrystallization into at least two product fractions;
IV) selecting at least one product fraction from those obtained in step III) and converting the at least one selected product fraction either by
IVa) hydrogenation to form the respective isomer of poly- or perfluoroalkyl sulfonic acid, or poly- or perfluoroalkyl carboxylic acid,
or
IVb) acidification to form the respective isomer of poly- or perfluoroalkyl sulfonic acid or perfluoroalkyl carboxylic acid.

2. Process according to claim 1, **characterized in that** step IIa) proceeds according to the
general scheme (1)
CₘF₂ₘ₊₁₋ₚHₚ-SO₂-X + Ar-OH → CₘF₂ₘ₊₁₋ₚHₚ-SO₂-OAr,
or
general scheme (2)
CₙF₂ₙ₊₁₋ₚHₚ-CO-Y + Ar-OH → CₙF₂ₙ₊₁₋ₚHₚ-CO-OAr,
wherein
Ar is a substituted or non-substituted aryl residue, preferably selected from the group consisting of substituted or non-substituted 1-naphthyl, substituted or non-substituted 2-naphthyl, heterocyclic naphthyls, in particular substituted or non-substituted 2-quinoliny or 8-quinolinyl, poly phenyls such as 4-phenylphenyl,
X is hydroxyl, fluorine or chlorine,
Y is hydroxyl, fluorine or chlorine,
m is an integer of at least 4, preferably 4 to 24, more preferably 4 to 18, even more preferably 4 to 12, in particular 8,
n is an integer of at least 3, preferably 3 to 23, more preferably 3 to 17, even more preferably 3 to 11, in particular 7,
p is 0 or 1 to ≤m, preferably 0 or 1 to 4.

3. Process according to claim 2, **characterized in that** the reactions are conducted in an organic solvent or solvent mixture, preferably organic ethers and/or organochlorine compounds, more preferably selected from the group consisting of dimethyl ether, diethyl ether, tetrahydrofurane, dioxane, dimethoxyethane, dichloromethane, chloroform and mixtures thereof, and in the presence of a base, preferably selected from the group consisting of triethyl amine and sodium hydride.

4. Process according to claim 1, **characterized in that** step IIb) proceeds according to the
general scheme (3)
CₘF₂ₘ₊₁₋ₚHₚ-SO₃H + NR¹R²R³ → CₘF₂ₘ₊₁₋ₚHₚ-SO₃⁻ N⁺HR¹R²R³
or
general scheme (4)
CₙF₂ₙ₊ᵢ₋ₚHₚ-CO₂H + NR¹R²R³ → CₙF₂ₙ₊₁₋ₚHₚ-CO₂⁻ N⁺HR¹R²R³,
wherein R¹, R² and R³ are, each independently of each other, hydrogen or an alkyl group with at least five carbon atoms, or R¹, R² and R³ together with the nitrogen can form a cycloalkyl amine or heterocyclic aromatic amine, and
m is an integer of at least 4, preferably 4 to 24, more preferably 4 to 18, even more preferably 4 to 12, in particular 8
n is an integer of at least 3, preferably 3 to 23, more preferably 3 to 17, even more preferably 3 to 11, in particular 7,
p is 0 or 1 to ≤m, preferably 0 or 1 to 4.

5. Process according to claim 4, **characterized in that** the amine NR¹R²R³ is selected from the group consisting of primary amines, secondary amines, tertiary amines, and mixtures thereof, the amine being particularly preferably selected from the group consisting of cyclohexyl amine, piperazine, N-phenyl-piperazine, pyridine and mixtures thereof and **in that** the reactions are conducted in an organic solvent, preferably selected from the group consisting of acetone, isopropanol and mixtures thereof.

6. Process according to any one of the preceding claims, **characterized in that**
- step IVa) is a hydrogenation with hydrogen gas in the presence of a hydrogenation catalyst, in particular palladium supported on charcoal or activated carbon, and the solvent for the reactions is an organic solvent, preferably selected from the group consisting of methanol, ethanol, n-propanol, iso-propanol, n-butanol, iso-butanol and mixtures thereof.
or
- step IVb) is an acidification with an inorganic acid or a mixture of inorganic acids, in particular hydrochloric acid and/or sulphonic acid.

7. Process according to any one of the preceding claims, **characterized in that** the product fraction selected in step IV) is a fraction containing or consisting mainly of a specific n-isomer.

8. Process according to any one of the preceding claims, **characterized in that** the isomer obtained in step IV) is either
perfluoro-n-octyl sulfonic acid, or
perfluoro-n-octyl carboxylic acid.

9. Perfluoroalkyl sulfonic acids of the general formula (Ia)
CₘF₂ₘ₊₁-SO₂-OH;
perfluoroalkyl carboxylic acids of the general formula (IIa)
CₙF₂ₙ₊₁-CO-OH;
aryl esters of perfluoroalkyl sulfonic acids of the general formula (IIIa)
CₘF₂ₘ₊₁-SO₂-O-Ar;
aryl esters of perfluoroalkyl carboxylic acids of the general formula (IVa)
CₙF₂ₙ₊₁-CO-O-Ar;
ammonium salts of perfluoroalkyl sulfonic acids of the general formula (Va)
CₘF₂ₘ₊₁-SO₂O⁻N+HR¹R²R³;
ammonium salts of perfluoroalkyl carboxylic acids of the general formula (VIa)
CₙF₂ₙ₊₁-COO⁻N⁺HR¹R²R³;
wherein
m is an integer of at least 4, preferably 4 to 24, more preferably 4 to 18, even more preferably 4 to 12, in particular 8,
n is an integer of at least 3, preferably 3 to 23, more preferably 3 to 17, even more preferably 3 to 11, in particular 7,
Ar is a substituted or non-substituted aryl residue, preferably selected from the group consisting of substituted or non-substituted 1-naphthyl, substituted or non-substituted 2-naphthyl, heterocyclic naphthyls, in particular substituted or non-substituted 2-quinoliny or 8-quinolinyl, poly phenyls such as 4-phenylphenyl,
R¹, R² and R³ are, each independently of each other, hydrogen or an alkyl group with at least five carbon atoms, or R¹, R² and R³ together with the nitrogen can form a cycloalkyl amine or heterocyclic aromatic amine,
**characterized in that** their isomerical purity is at least 97 % chromatographic purity, preferably at least 98 % more preferably at least 99 % and in particular at least 99.5 %.

10. Perfluoroalkyl sulfonic acids and perfluoroalkyl carboxylic acids, in particular according to claim 9, selected from the following:
- perfluoro-n-butyl sulfonic acid,
- perfluoro-n-pentyl sulfonic acid,
- perfluoro-n-hexyl sulfonic acid,
- perfluoro-n-heptyl sulfonic acid,
- perfluoro-n-octyl sulfonic acid,
- perfluoro-n-nonyl sulfonic acid,
- perfluoro-n-decyl sulfonic acid,
- perfluoro-n-undecyl sulfonic acid,
- perfluoro-n-dodecyl sulfonic acid,
- perfluoro-n-tridecyl sulfonic acid,
- perfluoro-n-tetradecyl sulfonic acid,
- perfluoro-n-pentadecyl sulfonic acid,
- perfluoro-n-hexadecyl sulfonic acid,
- perfluoro-n-heptadecyl sulfonic acid,
- perfluoro-n-octadecyl sulfonic acid,
- perfluoro-n-butyl carboxylic acid,
- perfluoro-n-pentyl carboxylic acid,
- perfluoro-n-hexyl carboxylic acid,
- perfluoro-n-heptyl carboxylic acid,
- perfluoro-n-octyl carboxylic acid,
- perfluoro-n-nonyl carboxylic acid,
- perfluoro-n-decyl carboxylic acid,
- perfluoro-n-undecyl carboxylic acid,
- perfluoro-n-dodecyl carboxylic acid,
- perfluoro-n-tridecyl carboxylic acid,
- perfluoro-n-tetradecyl carboxylic acid,
- perfluoro-n-pentadecyl carboxylic acid,
- perfluoro-n-hexadecyl carboxylic acid,
- perfluoro-n-heptadecyl carboxylic acid,
- perfluoro-n-octadecyl carboxylic acid;
and preferably selected from the following
- perfluoro-n-hexyl sulfonic acid,
- perfluoro-n-hexyl carboxylic acid,
- perfluoro-n-decyl sulfonic acid,
- perfluoro-n-octyl sulfonic acid,
- perfluoro-n-octyl carboxylic acid.
- perfluoro-n-decyl carboxylic acid,
- perfluoro-n-dodecyl sulfonic acid,
- perfluoro-n-dodecyl carboxylic acid;
and in particular selected from
- perfluoro-n-octyl sulfonic acid, and
- perfluoro-n-octyl carboxylic acid,
**characterized in that** their isomerical purity is at least 97 % chromatographic purity, preferably at least 98 % more preferably at least 99 % and in particular at least 99.5 %.

11. Polyfluoroalkyl sulfonic acids and polyfluoroalkyl carboxylic acids in particular according to claim 9 or 10, selected from the group consisting of the following: 2H,2H-perfluorooctanoic acid; 2H,2H-perfluorodecanoic acid; 2H,2H,3H,3H-perfluoroundecanoic acid; 2H,2H,3H,3H-perfluorododecanoic acid; 2H,2H,3H,3H-perfluorohexanoic acid; 2H,2H,3H,3H-perfluorooctanoic acid; 2H,2H,3H,3H-perfluorononanoic acid; 2H,2H,3H,3H-perfluorodecanoic acid; 2H-perfluoroethanoic acid; 2H- and 3H-perfluoropropanoic acid; 2H- and 4H-perfluorobutanoic acid; 2H- and 5H-perfluoropentanoic acid; 2H- and 6H-perfluorohexanoic acid; 2H- and 7H-perfluoroheptanoic acid; 2H- and 8H-perfluorooctanoic acid; 2H- and 9H-perfluorononanoic acid; 2H- and 10H-perfluorodecanoic acid; 2H- and 11H-perfluoroundecanoic acid; 1H,1H,2H,2H-perfluorobutanesulfonic acid; 1H,1H,2H,2H-perfluorohexanesulfonic acid; 1H,1H,2H,2H-perfluorooctanesulfonic acid; 1H,1H,2H,2H-perfluorodecanesulfonic acid; 1H,1H,2H,2H-perfluorododecanesulfonic acid and mixtures thereof.

12. Derivatives of isomerically pure poly- and perfluoroalkane sulfonic acids or carboxylic acids selected from the group consisting of
a) PFAS esters of telomeric alcohols;
b) PFAS allylic alcohol telomers;
c) PFAS telomeric olefins;
d) Poly- and Perfluoroalkyl N-alkylsulfonamides such as those of the following general formula:
CₘF₂ₘ₊₁₋ₚHₚ-SO₂-NHR,
in which R=H, methyl, ethyl, propyl or even higher alkyls;
e) Poly- and Perfluoroalkyl N-alkyl, N-hydroxyethylsulfonamides such as those of the following general formula:
CₘF₂ₘ₊₁₋ₚHₚ-SO₂-NR(CH₂CH₂OH),
in which R=H, methyl, ethyl, propyl or even higher alkyls;
f) Poly- and Perfluoroalkyl N-alkylsulfonylglycines;
g) Poly- and Perfluoroalkyl N-alkylsulfonylacrylates;
h) perfluoroalkane N-alkyl, N-hydroxyethylsulfonyl acrylates and methacrylates such as those of the following general formula: 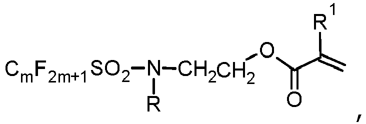 in which R=H, methyl, ethyl, propyl or even higher alkyls and R¹ = H, methyl;
i) PFAS allylic alcohols and telomeric olefins, preferably the following: 1H,1H,2H,3H-Perfluorohex-2-en-1-ol; 1H,1H,2H,3H-Perfluoronon-2-en-1-ol; 1H,1H,2H-Perfluoro-1-octene; 1H,1H,2H-Perfluoro-1-decene;
and mixtures thereof,
wherein
m is an integer of at least 4, preferably 4 to 24, more preferably 4 to 18, even more preferably 4 to 12, in particular 8;
p is 0 or 1 to ≤m, preferably 0 or 1 to 4.

13. Use of the poly- and perfluoroalkyl sulfonic acids or poly- and perfluoroalkyl carboxylic acids obtained in a process according to any one of claims 1 to 8 or according to claim 9 or according claim 10 or according to claim 11 or the derivatives according to claim 12 as reference materials or internal standards, preferably for analytical processes, in particular in the analysis and/or examination of poly- or perfluoroalkyl substances or products containing them.

14. Use according to claim 13, **characterized in that** the analytical processes, in particular analysis and/or examination of poly- or perfluoroalkyl substances or products containing them, is conducted with a method selected from the group consisting of HPLC, LC, GC, HRMS, ¹⁹F-NMR, ¹H-NMR, ¹³C-NMR and combinations thereof.
